# EUROPÄISCHE PATENTSCHRIFT

(11) **EP 0 670 299 B1**
(45) Veröffentlichungstag und Bekanntmachung des Hinweises auf die Patenterteilung: **14.10.1998**
(21) Anmeldenummer: 95102149.2
(22) Anmeldetag: 16.02.1995
(51) Int. Cl.: C07C 69/38, C07C 69/40, C07C 69/60, C07C 43/23, C09B 67/00

(54) **Entstaubungsmittel**
Dedusting agent
Agent de réduction de poussières

(30) Priorität: 01.03.1994 DE 4406630
(43) Veröffentlichungstag der Anmeldung: 06.09.1995
(73) Patentinhaber: BAYER AG, 51368 Leverkusen (DE)
(72) Erfinder: Brandt, Horst, Dr., D-51519 Odenthal (DE); Schulze, Hans, Dr., D-51061 Köln (DE); Nagel, Thomas, Dipl.-Ing., D-51467 Bergisch Gladbach (DE); Petroll, Hans-Werner, Dipl.-Ing., D-51515 Kürten-Herweg (DE); Herrmann, Udo, Dr., D-41541 Dormagen (DE)

(56) Entgegenhaltungen:
- EP-A- 0 023 638
- EP-A- 0 067 022
- EP-A- 0 200 388
- US-A- 4 238 577
- US-A- 4 562 096

## Beschreibung

Die Erfindung betrifft neue Entstaubungsmittel sowie diese enthaltende Feststoffpräparationen.

Feste, insbesondere pulverförmige Farbstoff-Präparationen neigen bei der Handhabung sowie bei Abfüll- oder Umfüllvorgängen bzw. beim Bereiten von Färbeflotten oder Farbstoffdruckpasten zum Stauben. Je nach Teilchenfeinheit bzw. Restfeuchte stauben diese mehr oder weniger ausgeprägt. Um Produktverluste und eine Kontamination der Arbeitsräume zu vermeiden oder vor allem um Arbeitshygiene für das Bedienungspersonal zu gewahrleisten, werden diese Pulver im allgemeinen mit entstaubend wirkenden Hilfsmitteln wie Mineralölen, allein oder in Kombination mit anionischen oder nichtionischen Tensiden, Phthalsäureestern, Silikonen oder Alkandiolen behandelt (vgl. z.B. DE-A 1 117 582, US-A 2 604 649, DE-A-834 237, DE-A-4 035 029). Um staubarme Präparationen zu erhalten, müssen die genannten Entstaubungszusätze dem Farbstoff in Mengen von ca. 2-10 % zugemischt werden. Die Anwendung dieser bekannten Produkte ist jedoch mit gewissen Nachteilen behaftet. So wird beispielsweise durch Mineralöle eine Klumpenbildung des Farbstoffpulvers gefördert. Weiterhin verschlechtern diese Mittel die Redispergier- und Lösungseigenschaften wie beispielsweise die für das Kaltfarbeverfahren wichtige Kaltwasserlöslichkeit. Weiterhin führt der Zusatz von Mineralölen häufig zur Bildung öliger Ablagerungen in den Färbeflotten, wodurch fleckige Warenbilder und Verunreinigungen der Färbemaschinen entstehen. Ein Zusatz von Tensiden wirkt dem zwar entgegen, bewirkt aber eine störende Schaumbildung und führt darüber hinaus bei Anwesenheit von Elektrolyten im Färbebad nicht zum Erfolg.

Die Nachteile der Silikone bestehen beispielsweise neben der Entmischung der Färbeflotten in Fleckenbildung auf der gefärbten Ware sowie in Anrandungen in den Färbeaggregaten.

Als Nachteile der Alkandiole sind beispielsweise ihre begrenzte Wirksamkeit zu nennen.

Da generell von staubenden Substanzen eine gesundheitsschädliche Gefährdung der damit umgehenden Personen ausgehen kann, werden heutzutage hohe Anforderungen an die Staubarmut von festen Stoffen, beispielsweise Farbstoffen, gestellt. Eine häufig angewandte Methode zur Bestimmung des Staubverhaltens von Farbstoffen ist beispielsweise in "Textilveredlung", 24 (1989), S. 277-290, beschrieben. Bei dieser Methode wird in einem luftdichten Stauberzeugungsgerät eine bestimmte Menge Farbstoffpulver schlagartig durch ein Fallrohr auf den Boden fallen gelassen. Nach 5 sec. wird der gebildete Staub durch Anlegen eines Vakuums abgesaugt und auf einem Filter gesammelt. Die Auswertung des Filters erfolgt visuell durch Vergleich nach dem 5-teiligen Graumaßstab nach ISO 105-A03. Dabei bedeutet ein Staubwert von 1, daß das Produkt stark staubend ist, ein Staubwert von 5, das kein Staubbelag auf dem Filter erkennbar ist. Nach heutigen Anforderungen sollten staubarme Farbstoff-Präparationen Staubwerte von mindestens 3 aufweisen. Für toxische Produkte sind Werte >4 dringend anzustreben.

In DE-A-2 656 406 und EP-A-200 388 wurde bereits versucht, dieses Problem mit Hilfe von Kondensationsprodukten aus Arylhydroxiden und Ethylenoxid zu lösen. Derartige Staubbindemittel weisen aber noch einige Nachteile auf, die es in der vorliegenden Anmeldung zu verbessern gilt.

Es wurden nun staubarme bzw. staubfreie Feststoff-Präparationen, insbesondere Farbstoffpräparationen gefunden, die zusätzlich zum Feststoff eine Verbindung der Formel (I) enthalten

R₁⁅A⁆Z⁅B⁆R₂ (I),

worin
- Z: für Z₁ oder Z₂ steht, wobei
- Z₁: einen bivalenten Rest der Formel 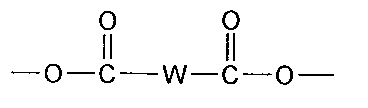 bedeutet, worin
- W: für eine direkte Bindung oder für eine C₁-C₃-Einheit steht, insbesondere für -CH₂-, -CH₂CH₂-, trans-(-CH=CH-) oder cis-(-CH=CH-) und
- Z₂: für unsubstituiertes Phenylendioxy oder Phenylenoxy steht,
- A und B: unabhängig voneinander jeweils einen bivalenten Rest aus bis zu 50 Ethylenoxid-Einheiten und/oder bis zu 50 Propylenoxid-Einheiten, die statistisch oder blockförmig angeordnet sind, bedeuten oder für eine direkte Bindung stehen, wobei die Alkylenoxygruppen so orientiert sind, daß das terminale Sauerstoffatom im Falle von A an R₁ und im Fall von B an R₂ gebunden ist, mit der Maßgabe, daß jeweils nur einer der bivalenten Reste A oder B die Bedeutung einer direkten Bindung annehmen kann und für den Fall, daß Z = Z₂ bedeutet, mindestens einer der beiden Reste A oder B Propylenoxid- und Ethylenoxid-Einheiten enthält, vorzugsweise in einem Verhältnis von 0,5:1 bis 10:1, insbesondere von 1:1 bis 4:1,
- R₁ und R₂: unabhängig voneinander Wasserstoff oder C₁-C₄-Alkyl, insbesondere Methyl, Ethyl, n-Propyl, iso-Propyl, n-Butyl und iso-Butyl bedeutet, wobei es sich bei dem Feststoff um ein Pflanzenschutzmittel, ein Pharmazeutikum oder einen Farbstoff handelt.

Bevorzugt sind Feststoffpräparationen, die zusätzlich zum Feststoff eine Verbindung der Formel (I) enthalten, die der Formel (II) entspricht 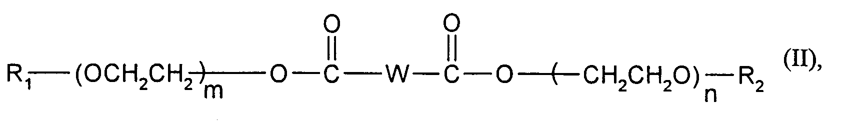 worin
- m und n: unabhängig voneinander für eine Zahl von 0 bis 10 stehen und die Summe aus m und n größer als Null ist,
- W: für eine direkte Bindung steht oder -CH₂-, -CH₂CH₂-, cis(-CH=CH-) oder trans(-CH=CH-) bedeutet und
- R₁ und R₂: unabhängig voneinander Methyl, Ethyl, n-Propyl, iso-Propyl, n-Butyl oder iso-Butyl bedeuten.

Verbindungen der Formel (II) lassen sich beispielsweise durch Ver- oder Umesterung der entsprechenden Dicarbonsäuren, -ester oder -anhydride von Oxalsäure, Malonsäure, Bernsteinsäure, Fumarsäure und Maleinsäure mit Alkoholen der Formel

R₁―(OCH₂CH₂)̵ₘOH

und/oder Alkoholen der Formel

R₂―(OCH₂CH₂)̵ₙOH

herstellen, wobei R₁, R₂, m und n die für die Verbindung der Formel (II) angegebene Bedeutung besitzen.

Weiterhin bevorzugt sind Feststoffpräparationen, die Verbindungen der Formel (I) enthalten, worin R₁ und R₂ Wasserstoff bedeuten, und Z für einen Phenylendioxy oder Phenyloxyrest steht.

Besonders bevorzugt sind Feststoffpräparationen, die zusätzlich zum Feststoff Verbindungen der Formel (I) enthalten, die denen der Formeln (III) und/oder (IV) entsprechen 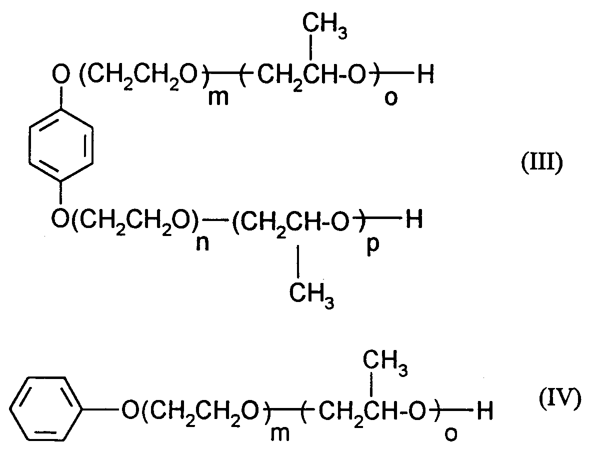 wobei
m, n, o und p unabhängig voneinander für eine Zahl von 0 bis 50 stehen und das Verhältnis von o : m 0,5 : 1 bis 10 : 1, vorzugsweise 1:1 bis 4:1, beträgt.

Die Verbindungen der Formeln (III) und (IV) können beispielsweise dadurch erhalten werden, daß man die entsprechenden Phenolverbindungen oder Hydrochinonverbindungen zunächst mit Ethylendioxid umsetzt und die Alkoxylierung anschließend mit Propylenoxid fortsetzt. Unter Katalyse mit Kaliumhydroxid in einem hochsiedenden Glykolether (Sdp. ca. 150°C) werden Ethylenoxid und Propylenoxid nacheinander in dem gewünschten Verhältnis angelagert.

Die Indices der Alkylenoxideinheiten m, n, o und p stellen statistische Mittelwerte dar.

Ganz besonders bevorzugt sind die erfindungsgemaß eingesetzten Entstaubungsmittel der Formeln (III) bzw. (IV), in denen m für eine Zahl von 2 bis 5, n für eine Zahl von 2 bis 5, o für eine Zahl von 1 bis 10 und p für eine Zahl von 1 bis 10 stehen.

Die Erfindung betrifft weiterhin Entstaubungsmittel der Formel (I), worin Z für Z₂ steht und die übrigen Bedeutungen so wie sie oben definiert sind sowie ihre bevorzugten Ausführungsformen wie sie für den Einsatz in den erfindungsgemäßen Feststoffpräparationen bereits oben beschrieben sind.

Die erfindungsgemäßen Feststoffpräparationen enthalten als Feststoff Pflanzenschutzmittel, Pharmazeutika, insbesondere jedoch Farbstoffe. Geeignete Farbstoffe sind beispielsweise Substantivfarbstoffe, kationische Farbstoffe, Reaktivfarbstoffe oder Dispersionsfarbstoffe sowie optische Aufheller, insbesondere jedoch Reaktivfarbstoffe und kationische Farbstoffe.

Die Feststoff-Präparationen, insbesondere Farbstoff-Präparationen, enthalten zusätzlich zum Feststoff im allgemeinen als Entstaubungsmittel 0,001 bis 10 Gew.-% der Verbindung der Formel (I), bezogen auf die gesamte Präparation. Ein höherer Anteil an Entstaubungsmittel ist zwar möglich, bietet allerdings in der Regel keinen Vorteil.

Die erfindungsgemäßen Feststoff-Präparationen setzen sich im allgemeinen wie folgt zusammen (bezogen auf die gesamte Präparation):
20 bis 90 Gew.-%, vorzugsweise 20 bis 80 Gew.-% Feststoff,
0,001 bis 10 Gew.-%, vorzugsweise 0,5 bis 6 Gew.-%, insbesondere 0,5 bis 3 Gew.-% der Verbindung der Formel (I).

Desweiteren können die Farbstoff-Präparationen noch übliche Zusätze in der üblichen Menge enthalten. Diese können beispielsweise Stellmittel, Salze, Restwasser, Puffer, Netzmittel, Dispergatoren, Tenside, Bindemittel oder andere sein.

Für Reaktivfarbstoffe sind beispielsweise als übliche und vorteilhafte Zusätze zu nennen: Kondensationsprodukte aus Naphthalin, Formaldehyd und Schwefelsäure oder aus Ditolylether, Formaldehyd und Na(HSO₃), Na₂SO₄, Puffer und hydrotrope Substanzen.

Für Dispersionsfarbstoffe sind beispielsweise als übliche Zusätze zu nennen: Dispergiermittel auf Basis von Ligninsulfonsäure, Kondensationsprodukte wie in Reaktivmischungen sowie Netzmittel.

Für Substantivfarbstoffe sind beispielsweise als übliche Zusätze zu nennen: Anorganische Salze, wasserlösliche Stärkeprodukte oder Wasserenthärtungsmittel.

Für kationische Farbstoffe sind beispielsweise als übliche Zusätze zu nennen: Anorganische Salze, wasserlösliche und -unlösliche Stärkeprodukte, anorganische Säuren, Komplexierungsmittel.

Die staubarmen Farbstoff-Präparationen kann man auf unterschiedliche Weise herstellen. So kann das Entstaubungsmittel beispielsweise einem Farbstoffslurry (Lösung oder Dispersion) zugesetzt werden und dann unter trocknenden Bedingungen wie beispielsweise durch Sprühtrocknung, Fließbetttrocknung oder Trocknung und Granulierung in die staubfreie Pulver- oder Granulatform überführt werden. Der Farbstoffslurry kann dabei vor, während oder nach der Entstaubungsmittelzugabe natürlich noch weiter behandelt werden. So ist beispielsweise eine Entsalzung von Reaktivfarbstoffen vor der Entstaubungsmittelzugabe mittels der Membrantechnik möglich. Eine Weiterbehandlung kann ebenfalls darin bestehen, den Slurry mit weiteren Zusätzen wie Stellmitteln, Salzen, Puffern usw. zu versehen.

Ausgehend von festen Farbstoffen wie Pulvern oder Granulaten kann die Zugabe des Entstaubungsmiffels in Pulvermischern, beispielsweise in Form einer wäßrigen Emulsion oder als Aerosol erfolgen. Desweiteren ist die Zugabe des Entstaubungsmittels in Form eines Sprühnebels in einem Fließbett-Trockner möglich, oder aber nach der Sprühtrocknung. So sind beispielsweise Granulate, erhalten nach dem Verfahren der Roll-, Misch- oder Sprühgranulation, die dem Abrieb und damit der Staubbildung unterliegen, nach dem genannten Verfahren, insbesondere durch Zu-mischen des Entstaubungsmittel staubarm bzw. staubfrei zu bekommen.

Die erfindungsgemäßen Präparationen, insbesondere die von wasserlöslichen oder wäßrig dispergierbaren Farbstoffen zeichnen sich insbesondere durch sehr gute Staubwerte (≥ 4) aus, und besitzen darüber hinaus weitere gewünschte Eigenschaften, wie sehr gute Lagerstabilitäten selbst bei 50°C über einen Zeitraum von 4 Wochen. Die erfindungsgemäßen Präparationen behalten ihre Löslichkeit bzw. ihre Lösebeständigkeit in wäßriger Lösung bzw. Elektrolytenlösung bei. Die nachfolgenden Beispiele verdeutlichen die Entstaubungseigenschaften. Die dabei verwendeten Entstaubungsmittel haben keinerlei störenden Einfluß auf die Löslichkeit des Farbstoffes beispielsweise in wäßriger Lösung oder unter Zusatz von Elektrolyten wie Na₂SO₄, NaCl oder Na₂CO₃.

### Beispiel 1

In einer Mischapparatur werden 98 g eines elektrolythaltigen Granulates des Farbstoffes der Formel in Form des Alkalisalzes 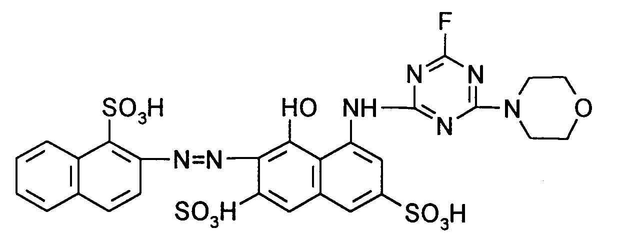 mit einem Farbstoffgehalt von 70 Gew.-% mit 2 g der Verbindung der Formel 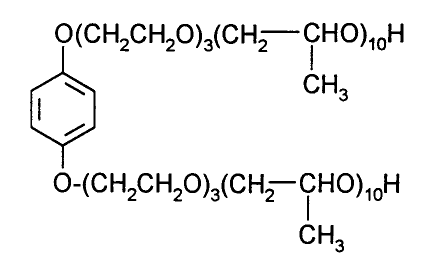 die durch Umsetzung von Hydrochinon mit zunächst 6 Mol-Äquivalenten Ethylenoxid und anschließend mit 20 Mol-Äquivalenten Propylenoxid erhalten wurde, zu einer homogenen Mischung vermischt. Die Indices der Alkylenoxid-Einheiten sind in der Formel idealisierte Werte. Die erhaltene Farbstoff-Präparation zeigt einen Staubwert von 4-5.

Die vorteilhafte Eigenschaft des nur sehr geringen Staubens der erfindungsgemäßen Farbstoff-Präparation bleibt auch bei Lagerung bei 50°C während 4 Wochen unverändert.

Die Farbstoff-Präparation wird zum Färben von Cellulosefasermaterialien, wie Baumwolle, eingesetzt, wobei das erfindungsgemäß verwendete Entstaubungsmittel die gute Qualität der Färbung nicht negativ beeinflußt.

### Beispiel 2

98 g des in Beispiel 1 verwendeten Ausgangs-Farbstoffgranulates werden mit 2 g der Verbindung der Formel 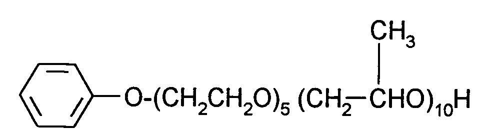 die aus der Umsetzung von Phenol mit zunächst 5 Mol-Äquivalenten Ethylenoxid und anschließend mit 10 Mol-Äquivalenten Propylenoxid erhalten wurde, in einer Mischapparatur homogen vermischt. Die Indices der Alkylenoxid-Einheiten der obigen Formel sind als idealisierte Werte zu verstehen. Die erhaltene erfindungsgemäße Farbstoff-Präparation zeigt einen Staubwert von 4, der auch nach Lagerung von 4 Wochen bei 50°C unverändert bleibt.

Die Farbstoff-Präparation wird zum Färben von Cellulosefasermaterialien, wie Baumwolle, eingesetzt, wobei das erfindungsgemaß verwendete Entstaubungsmittel die gute Qualität der Färbungen und Drucke in keiner Weise negativ beeinflußt.

### Beispiel 3

In einer Mischapparatur werden 98 g eines elektrolythaltigen Granulates des Farbstoffes der Formel in Form des Alkalisalzes 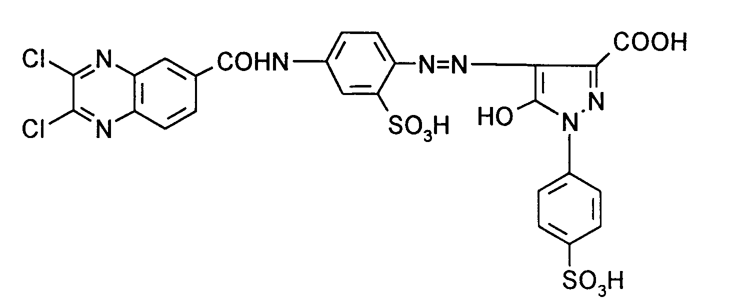 mit einem Farbstoffgehalt von 83 Gew.-% mit 2 g der Verbindung der Formel 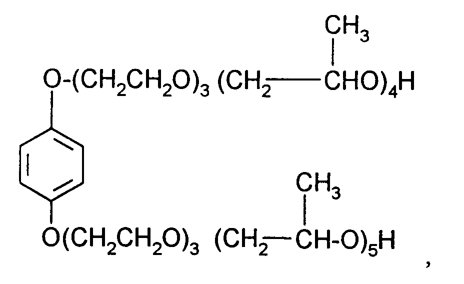 die aus der Umsetzung von Hydrochinon mit zunächst 6 Mol-Äquivalenten Ethylenoxid und anschließend mit 9 Mol-Äquivalenten Propylenoxid erhalten wurde, zu einer homogenen Mischung vermischt. Die Indices der AlkylenoxidEinheiten der obigen Formel sind als idealisierte Werte zu verstehen.

Die erhaltene Farbstoffpräparation ist gut löslich und zeigt einen Staubwert von 5. Diese vorteilhafte Eigenschaft des nur sehr geringen Staubens der erfindungsgemäßen Farbstoffpräparation bleibt auch bei einer Lagerung bei 50°C während 4 Wochen unverändert.

Die Farbstoffpräparation wird zum Färben von Baumwolle in farbstarken echten Tönen eingesetzt, wobei das erfindungsgemäß verwendete Entstaubungsmittel die gute Qualität der Färbungen und Drucke in keinerlei Weise negativ beeinflußt.

### Beispiel 4

Die Herstellung der Farbstoffpräparation erfolgt gemäß den Angaben aus Beispiel 3, jedoch wird statt des dort eingesetzten Entstaubungsmittels die gleiche Menge (2 g) an Maleinsäure-dimethyl-glykol-ester der Formel 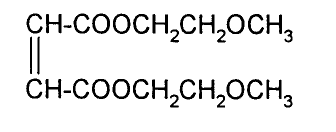 verwendet.

Die so erhaltene Farbstoffpräparation ist gut wasserlöslich und zeigt einen Staubwert von 5, der auch nach der Lagerung der Präparation bei 50°C während 4 Wochen unverändert bleibt.

### Beispiel 5

Eine Farbstoffpräparation wird mit dem Farbstoff und nach den Angaben aus Beispiel 3 hergestellt, jedoch wird statt des dort verwendeten Entstaubungsmittel 2 g an Maleinsäure-methyl-glykol-ethylglykol-ester der Formel 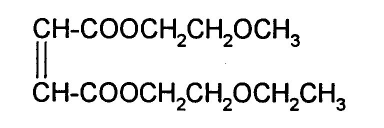 eingesetzt. Die so erhaltene Farbstoffpräparation ist sehr gut wasserlöslich und zeigt einen Staubwert von 4 bis 5, der auch nach Lagerung bei 50°C während 4 Wochen unverändert bleibt. Die erhaltenen Färbungen und Drucke von Baumwolle weisen die gleiche hohe Qualität auf wie die aus Beispiel 3.

### Beispiel 6

47 g eines Pulvers des Farbstoffes der Formel 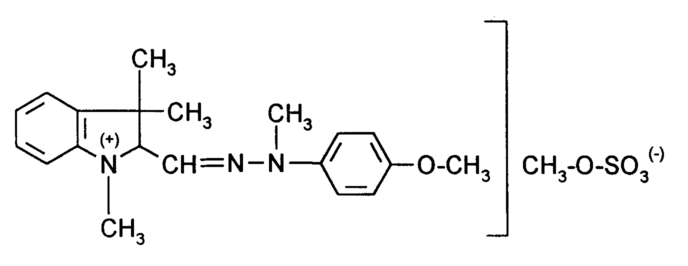 werden mit
52 g Natriumsulfat und 1 g Maleinsäure-methylglykol-ethylglykol-ester der Formel 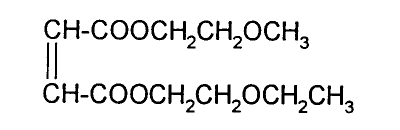 gemahlen und homogen gemischt.
Man erhält eine gut entstaubte Farbstoff-Präparation mit guter Wasserlöslichkeit. Die erfindungsgemäße Farbstoffmischung bleibt auch nach einer Lagerung von über 4 Wochen sowohl bei Raumtemperatur als auch bei einer Lagertemperatur von 50°C unverändert.

### Beispiel 7

47 g eines Farbstoffpulvers aus Beispiel 6 werden mit 52 g Natriumsulfat und 1 g Maleinsäure-di-methyl-di-glykol-ester der Formel 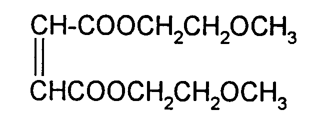 homogen gemischt. Die erhaltene Farbstoff-Präparation ist gut entstaubt und zeigt auch nach 4 Wochen Lagerung bei Raumtemperatur und bei 50°C einen unverändert stabilen Staubwert. Die erfindungsgemäß eingesetzten Entstaubungsmittel in Beispiel 6 und 7 besitzen keinen negativen Einfluß auf die Wasserlöslichkeit sowie Färbung in Polyacrylnitrilfasern. Zum Vergleich eingesetzte Öl/Emulgator-Mischungen ergaben bei identischem Einsatz ein schlechteres Staubverhalten.

### Beispiel 8

76 g des Pulverfarbstoffes der Formel 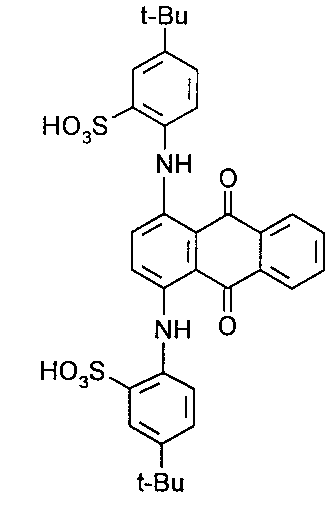 werden mit
20 g Dextrin und 4 g Maleinsäure-methyl-butyl-di-glykolester der Formel 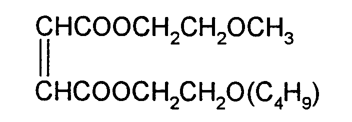 innig vermischt. Man erhält eine gering staubende Farbstoffmischung, die gut wasserlöslich ist. Nach 4 Wochen Lagerung bei Raumtemperatur, aber auch bei 50°C zeigt die erfindungsgemäß entstaubte Farbstoffmischung unveränderte Staubwerte.

### Beispiel 9

76 g eines Farbstoffpulvers aus Beispiel 8 werden mit 20 g Dextrin und 4 g Maleinsäure-methylglykol-ethylglykol-ester der Formel 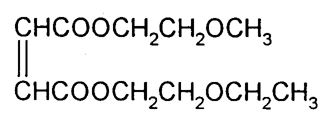 homogen in einer Mischapparatur gemischt.

Die erhaltene Farbstoff-Präparation ist gut wasserlöslich, gering staubend und selbst nach einer Lagerung bei 50°C nach 4 Wochen staubwertstabil.

Die zum Vergleich eingesetzten Öl/Emulgatormischungen ergaben bei identischem Einsatz deutlich schlechte Staubwerte.

### Beispiel 10

96 g eines sprühgetrockneten Farbstoffes, enthaltend 49,2 % des handelsüblichen Natriumsalzes eines Naphthalinsulfonsäure-Formaldehyd-Kondensationsproduktes, 8 % Restfeuchte und 42,8 % des Farbstoffes der Formel 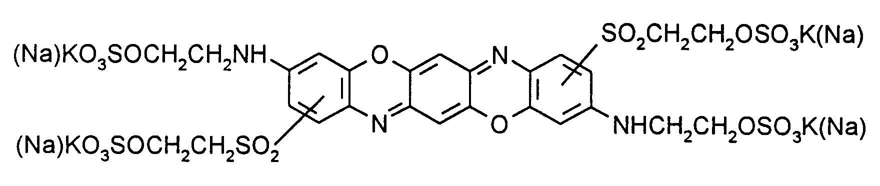 werden mit 4 g Maleinsäure-methyl-butylglykol-ester der Formel 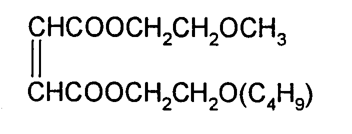 homogen gemischt.
Man erhält eine sehr gut entstaubte Farbstoffmischung, die selbst nach 4 Wochen Lagerung bei 60°C staubfrei bleibt. Die anwendungstechnischen Eigenschaften dieser entstaubten Farbstoff-Präparation (Löslichkeit, Färbung auf Cellullosefasern) bleiben unverändert.

### Beispiel 11

In einer Mischapparatur werden 99 g des eingestellten Reaktivfarbstoffs (mit 57 % Farbstoff 4,5 % Puffer, 29 % eines Kondensationsproduktes aus Naphthalin, Formaldehyd und Schwefelsäure sowie 8 % Restfeuchte) der Formel 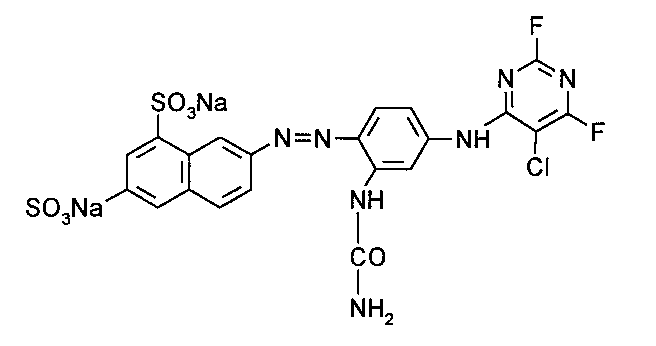 und 1 g der entstaubend wirkenden Verbindung
(CH₂-COOCH₂CH₂-OCH₂CH₂OCH₃)₂
vermischt.

Man erhält eine entstaubte Präparation mit einem Staubbereich von 5 und guter Wasserlöslichkeit, die in einer Klotzflotte keine Anrandungen ergibt.

Derselbe Reaktivfarbstoff ergibt mit Mineralöl versetzt nur mäßige Staubwerte verbunden mit obigen Abscheidungen in der Färbeflotte.

## Patentansprüche

1. Feststoffpräparationen enthaltend zusätzlich zum Feststoff Verbindungen der Formel (I)
R₁⁅A⁆Z⁅B⁆R₂ (I),
worin
Z für Z₁ oder Z₂ steht, wobei
Z₁ einen bivalenten Rest der Formel 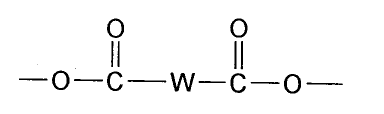 bedeutet, worin
W für eine direkte Bindung oder für eine C₁-C₃-Einheit steht, insbesondere für -CH₂-, -CH₂CH₂-, trans-(-CH=CH-) oder cis-(-CH=CH-) und
Z₂ für unsubstituiertes Phenylendioxy oder Phenylenoxy steht,
A und B unabhängig voneinander jeweils einen bivalenten Rest aus bis zu 50 Ethylenoxid-Einheiten und/oder bis zu 50 Propylenoxid-Einheiten, die statistisch oder blockförmig angeordnet sind, bedeuten oder für eine direkte Bindung stehen, wobei die Alkylenoxygruppen so orientiert sind, daß das terminale Sauerstoffatom im Falle von A an R₁ und im Fall von B an R₂ gebunden ist, mit der Maßgabe, daß jeweils nur einer der bivalenten Reste A oder B die Bedeutung einer direkten Bindung annehmen kann und für den Fall, daß Z = Z₂ bedeutet, mindestens einer der beiden Reste A oder B Propylenoxid- und Ethylenoxid-Einheiten enthält,
R₁ und R₂ unabhängig voneinander Wasserstoff oder C₁-C₄-Alkyl, insbesondere Methyl, Ethyl, n-Propyl, iso-Propyl, n-Butyl und iso-Butyl bedeutet,
wobei es sich bei dem Feststoff um ein Pflanzenschutzmittel, ein Pharmazeutikum oder einen Farbstoff handelt.

2. Feststoffpräparationen gemäß Anspruch 1, enthaltend zusätzlich zum Feststoff Verbindungen der Formel (II) 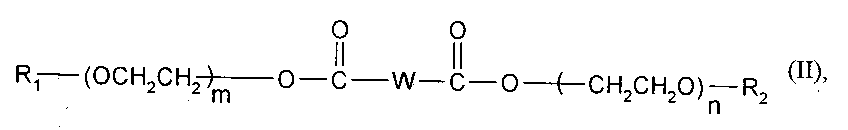 worin
m und n unabhängig voneinander für eine Zahl von 0 bis 10 stehen und die Summe aus m und n größer als Null ist,
W für eine direkte Bindung steht oder -CH₂-, -CH₂CH₂-, cis(-CH=CH-) oder trans(-CH=CH-) bedeutet und
R₁ und R₂ unabhängig voneinander Methyl, Ethyl, n-Propyl, iso-Propyl, n-Butyl oder iso-Butyl bedeuten.

3. Feststoffpräparationen gemäß Anspruch 1, enthaltend zusätzlich zum Feststoff Verbindungen der Formel (I), worin R₁ und R₂ Wasserstoff bedeuten, und Z für einen Phenylendioxy- oder Phenyloxyrest steht.

4. Feststoffpräparationen gemäß Anspruch 1, enthaltend zusätzlich zum Feststoff Verbindungen der Formeln (III) und/oder (IV) 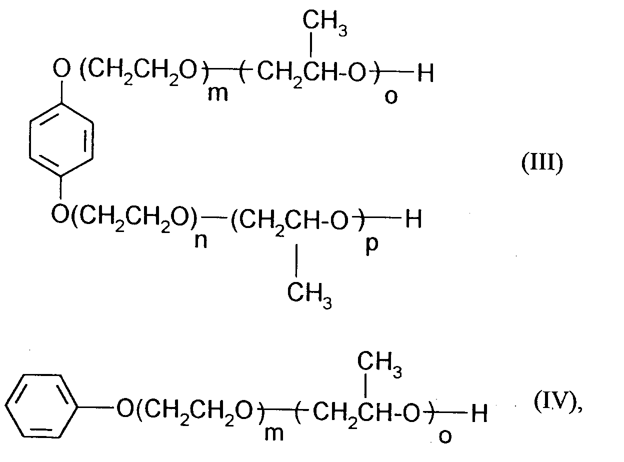 wobei
m, n, o und p unabhängig voneinander für eine Zahl von 0 bis 50 stehen, und das Verhältnis von o : m 0,5 bis 10:1 beträgt.

5. Feststoff-Präparationen gemäß Anspruch 1, dadurch gekennzeichnet, daß sie 0,001 bis 10 Gew.-% einer Verbindung der Formel (I), bezogen auf die gesamte Präparation, enthalten.

6. Feststoffpräparationen gemäß Anspruch 1, enthaltend
20 bis 90 Gew.-%, vorzugsweise 20 bis 80 Gew.-% eines Feststoffs und
0,001 bis10 Gew.-%, vorzugsweise 0,5 bis 6 Gew.-% einer Verbindung der Formel (I).

7. Verbindungen der Formel (I)
R₁⁅A⁆Z⁅B⁆R₂ (I),
worin
Z für Z₂ steht, wobei
Z₂ für unsubstituiertes Phenylendioxy oder Phenylenoxy steht,
A und B unabhängig voneinander jeweils einen bivalenten Rest aus bis zu 50 Ethylenoxid-Einheiten und/oder bis zu 50 Propylenoxid-Einheiten, die statistisch oder blockförmig angeordnet sind, bedeuten oder für eine direkte Bindung stehen, wobei die Alkylenoxygruppen so orientiert sind, daß das terminale Sauerstoffatom im Falle von A an R₁ und im Fall von B an R₂ gebunden ist, mit der Maßgabe, daß jeweils nur einer der bivalenten Reste A oder B die Bedeutung einer direkten Bindung annehmen kann und für den Fall, daß Z = Z₂ bedeutet, mindestens einer der beiden Reste A oder B Propylenoxid- und Ethylenoxid-Einheiten enthält,
R₁ und R₂ unabhängig voneinander Wasserstoff oder C₁-C₄-Alkyl, insbesondere Methyl, Ethyl, n-Propyl, iso-Propyl, n-Butyl und iso-Butyl bedeutet.

8. Verbindungen gemäß Anspruch 7 der Formel (I), worin R₁ und R₂ Wasserstoff bedeuten.

9. Verbindungen gemäß Anspruch 7 der Formel (III) und/oder (IV) 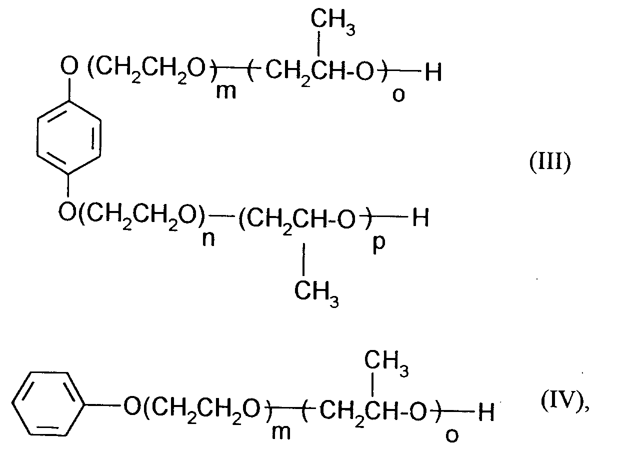 wobei
m, n, o und p unabhängig voneinander für eine Zahl von 0 bis 50 stehen, und das Verhältnis von o : m 0,5 bis 10:1 beträgt.

10. Verwendung der Verbindungen gemäß Anspruch 7 als Entstaubungsmittel.

11. Verfahren zum Färben, dadurch gekennzeichnet, daß man eine Feststoffpräparation gemäß Anspruch 1 verwendet.

## Claims

1. Solid preparations containing, in addition to the solid, compounds of the formula (I)
R₁⁅A⁆Z⁅B⁆R₂ (I),
in which
Z represents Z₁ or Z₂ where
Z₁ denotes a bivalent radical of the formula 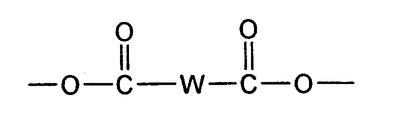 in which
W represents a direct bond or a C₁-C₃ unit, in particular -CH₂-, -CH₂CH₂-, trans(-CH=CH-) or cis-(-CH=CH-) and
Z₂ represents unsubstituted phenylenedioxy or phenyleneoxy,
A and B each denote, independently of one another, a bivalent radical consisting of up to 50 ethylene oxide units and/or up to 50 propylene oxide units arranged randomly or in blocks, or represent a direct bond where the alkyleneoxy groups are oriented in such a manner that the terminal oxygen atom of A is attached to R₁ and that of B is attached to R₂, with the proviso that in each case only one of the bivalent radicals A or B may adopt the meaning of a direct bond and in the case where Z denotes Z₂ at least one of the two radicals A or B contains propylene oxide and ethylene oxide units,
R₁ and R₂ denote, independently of one another hydrogen or C₁-C₄-alkyl, in particular methyl, ethyl, n-propyl, iso-propyl, n-butyl and iso-butyl,
the solid being a crop protection agent, a pharmaceutical or a dyestuff.

2. Solid preparations according to Claim 1 containing, in addition to the solid, compounds of the formula (II) 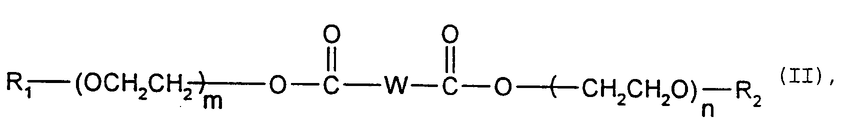 in which
m and n represent, independently of one another, 0 to 10 and the sum of m and n is greater than zero,
W represents a direct bond or -CH₂-, -CH₂CH₂-, cis(-CH=CH-) or trans(-CH=CH-) and
R₁ and R₂ denote, independently of one another, methyl, ethyl, n-propyl, iso-propyl, n-butyl or iso-butyl.

3. Solid preparations according to Claim 1 containing, in addition to the soid, compounds of the formula (I), in which R₁ and R₂ denote hydrogen and Z represents a phenylenedioxy or phenyloxy radical.

4. Solid preparations according to Claim 1 containing, in addition to the solid, compounds of the formulae (III) and/or (IV) 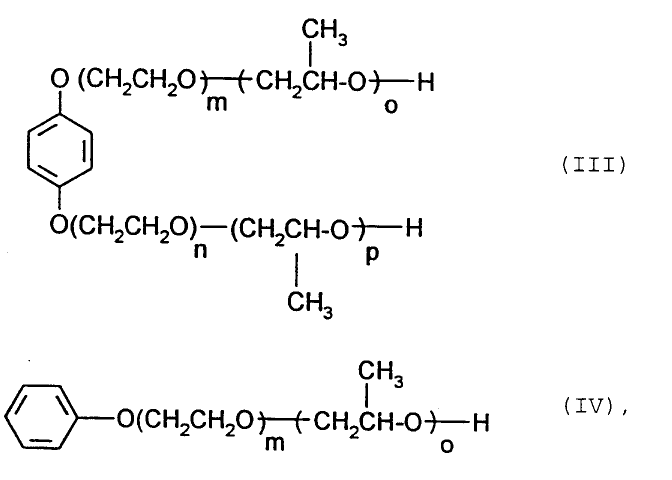 wherein
m, n, o and p represent, independently of one another, 0 to 50 and the o/m ratio is 0.5 to 10:1.

5. Solid preparations according to Claim 1, characterized in that they contain 0.001 to 10% by weight of a compound of the formula (I), relative to the overall preparation.

6. Solid preparations according to Claim 1, containing
20 to 90% by weight, preferably 20 to 80% by weight, of a solid and 0.001 to 10% by weight, preferably 0.5 to 6% by weight, of a compound of the formula (I).

7. Compounds of the formula (I)
R₁⁅A⁆Z⁅B⁆R₂ (I),
in which
Z represents Z₂ where
Z₂ represents unsubstituted phenylenedioxy or phenyleneoxy,
A and B each denote, independently of one another, a bivalent radical consisting of up to 50 ethylene oxide units and/or up to 50 propylene oxide units arranged randomly or in blocks, or represent a direct bond where the alkyleneoxy groups are oriented in such a manner that the terminal oxygen atom of A is attached to R₁ and that of B is attached to R₂, with the proviso that in each case only one of the bivalent radicals A or B may adopt the meaning of a direct bond and in the case where Z denotes Z₂ at least one of the two radicals A or B contains propylene oxide and ethylene oxide units,
R₁ and R₂ denote, independently of one another hydrogen or C₁-C₄-alkyl, in particular methyl, ethyl, n-propyl, iso-propyl, n-butyl and iso-butyl.

8. Compounds according to Claim 7 of the formula (I) in which R₁ and R₂ denote hydrogen.

9. Compounds according to Claim 7 of the formula (III) and/or (IV) 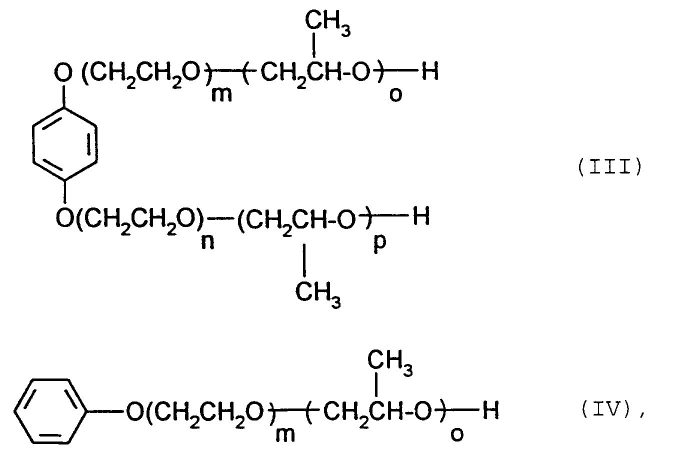 wherein
m, n, o and p represent, independently of one another, 0 to 50 and the o/m ratio is 0.5 to 10:1.

10. Use of the compounds according to Claim 7 as dust-proofing agent.

11. Process for dyeing, characterized in that a solid preparation according to Claim 1 is used.

## Revendications

1. Compositions de matières solides contenant, en plus de la matière solide, des composés de formule
R_{f}⁅A⁆Z⁅B⁆R₂ (I),
dans laquelle
Z représente Z₁ ou Z₂,
Z₁ représente un radical bivalent de formule 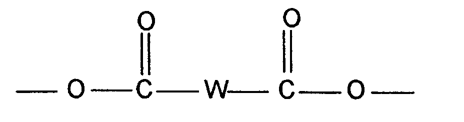
dans laquelle
W représente une liaison directe ou un motif en C₁-C₃, plus spécialement un motif -CH₂-, -CH₂CH₂-, trans-(-CH=CH-) ou cis-(-CH=CH-) et
Z₂ représente un groupe phénylènedioxy ou phénylèneoxy non substitué,
A et B représentent chacun, indépendamment l'un de l'autre, un radical bivalent de motifs d'oxyde d'éthylène, jusqu'à 50, et/ou de motifs d'oxyde de propylène, jusqu'à 50, en disposition statistique ou séquencée, ou bien une liaison directe, les groupes alkylèneoxy étant orientés en sorte que l'atome d'oxygène terminal de A est relié à R₁ et l'atome d'oxygène terminal de B est relié à R₂, sous réserve qu'un seul des symboles A ou B peut représenter une liaison directe, et lorsque Z = Z₂, au moins un des deux radicaux A ou B contient des motifs d'oxyde de propylène et d'oxyde d'éthylène,
R₁ et R₂ représentent chacun, indépendamment l'un de l'autre, l'hydrogène ou un groupe alkyle en C₁-C₄, plus spécialement méthyle, éthyle, n-propyle, isopropyle, n-butyle et isobutyle,
la matière solide étant un produit phytosanitaire, un produit pharmaceutique ou un colorant.

2. Compositions de matières solides selon la revendication 1, contenant, en plus de la matière solide, des composés de formule (II)
R₁―(OCH₂CH₂―)ₘ―O―C―W―C―O(̵CH₂CH₂O)ₙ―R₂ (II)
dans laquelle
m et n sont égaux chacun, indépendamment l'un de l'autre, à un nombre de 0 à 10 et la somme de m et de n est supérieure à 0,
W représente une liaison directe ou -CH₂-, -CH₂CH₂-, cis-(-CH=CH-) ou trans-(-CH=CH-) et
R₁ et R₂ représentent chacun, indépendamment l'un de l'autre, un groupe méthyle, éthyle, n-propyle, isopropyle, n-butyle ou isobutyle.

3. Compositions de matières solides selon la revendication 1, contenant, en plus de la matière solide, des composés de formule I dans laquelle R₁ et R₂ représentent l'hydrogène et Z un groupe phénylènedioxy ou phényloxy.

4. Compositions de matières solides selon la revendication 1, contenant, en plus de la matière solide, des composés de formules III et/ou IV : 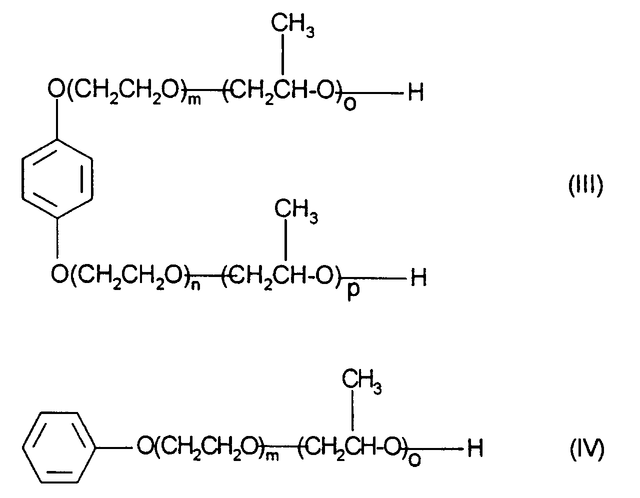 dans lesquelles
m, n, o et p représentent chacun, indépendamment les uns des autres, un nombre de 0 à 50, avec un rapport o : m qui va de 0,5:1 à 10:1.

5. Compositions de matières solides selon la revendication 1, caractérisées en ce qu'elles contiennent de 0,001 à 10 % en poids d'un composé de formule (I) sur son poids total.

6. Compositions de matières solides selon la revendication 1, contenant
20 à 90 % en poids, de préférence 20 à 80 % en poids d'une matière solide et 0,001 à 10 % en poids, de préférence 0,5 à 6 % en poids d'un composé de formule (I).

7. Composés de formule (I)
R_{f}⁅A⁆Z⁅B⁆R₂ (I),
dans laquelle
Z représente Z₂,
Z₂ représente un groupe phénylènedioxy ou phénylèneoxy non substitué,
A et B représentent chacun, indépendamment l'un de l'autre, un radical bivalent consistant en motifs d'oxyde d'éthylène, jusqu'à 50, et/ou en motifs d'oxyde de propylène, jusqu'à 50, qui sont en disposition statistique ou séquencée, ou bien une liaison directe, les groupes alkylèneoxy étant orientés en sorte que l'atome d'oxygène terminal de A est relié à R₁ et l'atome d'oxygène terminal de B est relié à R₂, sous réserve qu'un seul des symboles A ou B peut représenter une liaison directe et que lorsque Z = Z₂, au moins un des deux radicaux A ou B contient des motifs d'oxyde de propylène et d'oxyde d'éthylène,
R₁ et R₂ représentent chacun, indépendamment l'un de l'autre, l'hydrogène ou un groupe alkyle en C₁-C₄, plus spécialement méthyle, éthyle, n-propyle, isopropyle, n-butyle et isobutyle.

8. Composés selon la revendication 7, répondant à la formule (I) dans laquelle R₁ et R₂ représentent l'hydrogène.

9. Composés selon la revendication 7, répondant aux formules (III) et/ou (IV). 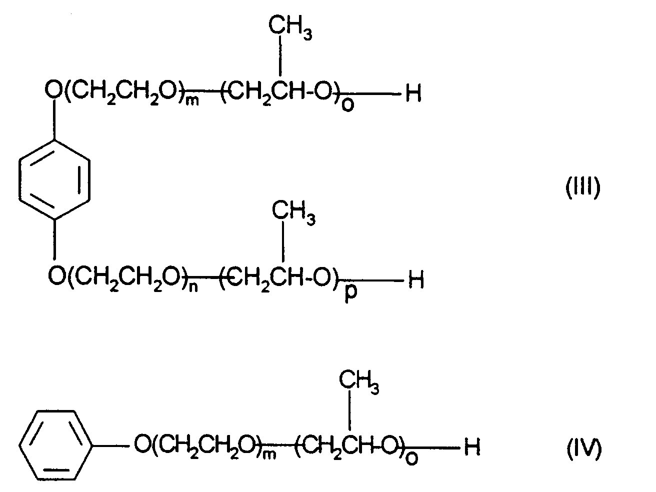 dans lesquelles
m, n, o et p représentent chacun, indépendamment les uns des autres, un nombre de 0 à 50, avec un rapport o : m qui va de 0,5:1 à 10:1.

10. Utilisation des composés selon la revendication 7 en tant qu'agents dépoussiérants.

11. Procédé de teinture, caractérisé en ce que l'on utilise une composition de matières solide selon la revendication 1.
